# EUROPEAN PATENT APPLICATION

(11) **EP 2 039 291 A1**
(43) Date of publication of application: **25.03.2009**
(21) Application number: 07018303.3
(22) Date of filing: 18.09.2007
(51) Int. Cl.: A61B 5/085

(54) **Method for monitoring respiration activity, system for compensation of motion artefacts caused by respiration activity and their use**

(71) Applicant: SIEMENS AKTIENGESELLSCHAFT, 80333 München (DE)
(72) Inventor: The designation of the inventor has not yet been filed

(57) **Abstract**

Method (100) for monitoring respiration activity of a subject (10) and system (50) for compensation of motion artefacts within electrophysiological data caused by respiration activity. The method (100) comprising
- (102) attaching a first electrode (12) on a surface (14) of said subject (10),
- (104) locating a second electrode (16) inside a thorax (11) of said subject (10),
- (106) applying an ac voltage (18) to one of said electrodes (12,16),
- (108) measuring an impedance (20) between said first electrode (12) and second electrodes (16), and
- (110) observing a change of impedance (20) caused by said respiration activity.

## Description

This invention relates to a method for monitoring respiration activity, a system for compensation of motion artefacts caused by respiration activity and their use.

During minimal invasive procedures within a thoracic region of a subject it is important to image for instance the anatomy of heart and lung tissue. These images have to have as high resolution as possible in order to illustrate detailed anatomic structures. Due to motion caused by respiration activity it is therefore often required that the breathing subject holds his/her breath during the acquisition of data to acquire high quality image data. However, during acquisition procedures lasting more than 30 seconds, or when it is not feasible to interrupt the patient's respiration, the breath-holding technique can not be applied. For example some electrophysiological data such as electrocardiographics (ECGs) and a stroke volume of the heart measured by a pacemaker is added to a respiratory pattern. Under these circumstances it is necessary to implement an actual compensation for motions artefacts caused by respiration activity.

Additionally it is of great importance also to monitor respiration activity, for example, during invasive procedures. A position of a medical device can be estimated by measuring the distance to at least two reference body surface electrodes. Since these distances will change due to respiration activity, the estimated position of the medical device will be exposed to motion artefacts especially a motion of the chest with the reference body surface electrodes caused by these activities.

There are for instance monitoring systems and electro anatomic mapping systems available that estimate the respiration activity by measuring of impedance changes between two body surface electrodes. Due to inflation of air in the patients lungs, the impedance will increase during inspiration and decrease during expiration. However, a quantity of lung tissue between the body surface electrodes is very limited so the electrical signal penetrating the tissue is not exposed to easily measured variations. Therefore this method is known to be quite unreliable as referenced in George B. Moody, Roger G. Mark, Andrea Zoccola, and Sara Mantero: Derivation of Respiratory Signals from Multi-lead ECGs. Computers in Cardiology 1985, vol. 12, pp. 113-116.

Available systems estimate respiration activity by impedance changes between certain body surface electrodes. This approach is exposed to limitations caused by very limited lung tissue penetrated by the electrical signal.

The US 2006/0241512 A1 discloses a method for thoracic impedance detection, comprising sensing an interelectrode impedance with an electrode implanted in a subject thorax and electrodes that are located on an outer surface of the subject's heart. This electrode configuration is sensing intrinsic electrical heart signals and a respiration signal for diagnostic purposes only.

It is one object of the invention to monitoring respiration activity that overcomes the above mentioned limitations.

A better approach for monitoring respiration activity is to measure impedance changes between an intracardiac electrode and a body surface electrode. A line defined by the intracardiac electrode and the body surface electrode crosses a lung tissue providing a higher electrical penetration. This electrode configuration can be easily obtained by attaching the body surface electrodes on a chest of the breathing subject. Such an attaching of the body surface monopolar electrode is similar to precordial leads by Wilson. By applying an AC voltage to one or several intracardiac electrodes, the current or voltage drop can be measured by one or several body surface electrodes.

One of the body surface electrodes can define the lung tissue crossing line with maximum crossing of the lung tissue. The measured impedances between one of the intracardiac electrodes and the body surface electrodes can be processed by frequency filtering. A further example of a method comprises applying of several voltages with several frequencies to several intracardiac electrodes. This example makes more than one measuring of impedance in one respiratory phase of the subject available. Several frequencies would distinguish each impedance measurement in this case to analyze the impedances individually.

The change of impedance might be observed by calculating a weighted sum of all impedance measurements. Portions of the lung tissue with a suitable change of volume caused by respiration activity could be higher weighted. A right lung portion, for example, which is not pathological changed, could be crossed by one line from an intracardiac electrode to a second body surface electrode attached on the right chest wall of the subject.

The monitoring of respiration activity comprises at least a signal indicating the respiratory phase. If the observed change of impedance is negative a respiratory phase signal for the expiration occurs. The negative or positive change of impedance can be displayed to inform a physician.

It is another object of the invention to accomplish a compensation of motion artefacts caused by respiration activity within electrophysiological data efficiently.

A system for compensation of these artefacts from ECGs is known by referenced article. That system comprises
- means for electrical acquiring of said electrophysiological data of a subject, comprising electrodes on the subject's body, and
- detecting means for motions of the subject's chest, whereby said electrophysiological data is compensated with said motion of the chest.

For detecting motions of the chest the volume of air needs to be estimated in real time so that the delta change of volume, i.e. inhaled or exhaled airflow, can be correlated to chest motion. Once the motion of the chest and each organ is estimated, algorithms for motion compensation can be implemented.

The system of the invention monitors the respiration activity of the subject by observing the impedance change between the intracardiac electrodes and an electrode for acquiring the electrophysiological data. The change of lung volume could be derived from the observed change of impedance by computer assisted circuitry. That derived change of lung volume can be correlated with the chest motion by a computer circuitry.

It is a further object of the invention to disclose several applications for the above mentioned methods of the invention.

Said methods can be used for compensation of respiration motion artefacts during image acquisition where breath holding is not feasible.

Said methods can be used for deriving, displaying, and monitoring a respiration rate during operational procedures or during intensive care. The respiration rate can easily be derived from information about lung volume changes and displayed to staff in an operation room. With the monitoring of respiration activity in real time, a compensation for these motion artefacts can be implemented.

Said methods can be used for compensation of respiration motion artefacts in measured invasive pressure waveforms. Such pressure waveforms comprise a blood pressure within a blood vessel, which are measured by a special catheter inside the vessel. This measure is influenced by respiration activity and could be corrected for calculation of flow rates of blood for example in an arch of the aorta.

It is still another object of the invention to compensate respiration motion artefacts during estimating the position of a medical device such as a catheter, which is tracked by measuring distances between two electrodes on a subject's body and the medical device. The respiration motion artefacts cause a motion of the reference body electrodes indicating another position of the medical device. The movement of the electrodes change the distances without moving the medical device. A range of movement of the electrodes can be calculated with the above mentioned methods. The correlated motion of the chest could be utilized for calculating that range.

In the accompanying drawings several embodiments of the invention are illustrated. With reference to the drawings, by way of example, the invention will be described in detail but not by way of limitation. Same numerals in the drawings represent the same technical features. The drawings are not drawn to scale.
- Fig.1: illustrates a schematic flowchart of a first example of the invention,
- Fig.2: illustrates a system according to the invention, and
- Fig.3: illustrates a use of a second example according to the method of the invention.

The following description of embodiments refers to the accompanying drawings.

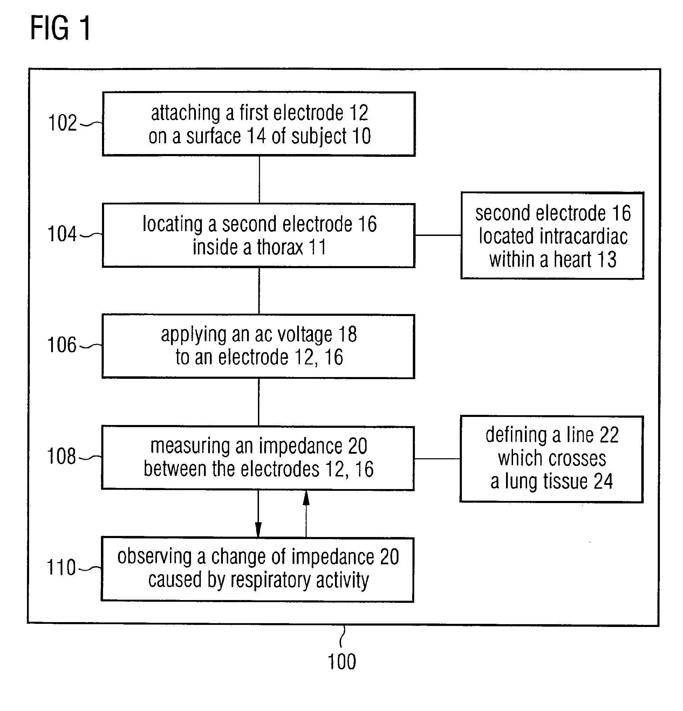

Figure 1 shows a schematic flowchart of a first embodiment of a method 100 for monitoring respiration activity of a subject 10. A thoracic impedance measure is done with an electrode configuration by 102 attaching a first electrode 12 on a surface 14 of said subject 10 and 104 locating a second electrode 16 inside a thorax 11 of said subject 10. Said second electrode 16 is at least one intracardiac electrode located within a heart 13 of said subject 10. To one of said second electrodes 16 an ac voltage 18 is applied 106. For measuring 108 an impedance 20 between said first electrode 12 and second electrodes 16, for example, the voltage 18 at the first surface body electrode 12 is registered in connection with its frequency. Under the assumption of linearity resistance of a tissue between the electrodes 12 and 16 a loss of the voltage 18 is correlated to the length of a line 22. That line 22 is defined by the first electrode 12 and the intracardiac electrode 16, which crosses lung tissue 24 of said subject 10. During respiration activity the volume of the lung will change. Thus the length of the line 22 and also the electric penetration of the lung tissue 24 would be reduced by expiration. To observe 110 such a change of impedance 20 caused by said respiration activity, said measuring 108 of impedance 20 is repeated several times or will be observed by comparing said impedance 20 with a thoracic impedance.

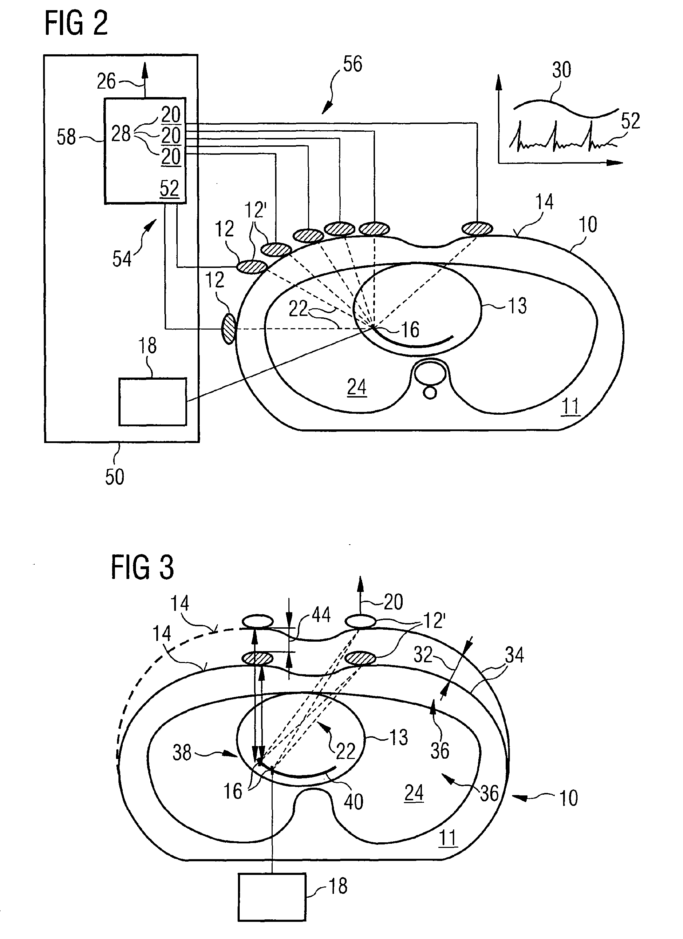

With said observed change of impedance 20 and therefore a change of the length of the line 22 a changing location of viscera organs could be tracked. Thus during image acquisition the changing location of e.g. the heart 13, a blood vessel, a liver or a kidney could be motion corrected for significant better resolution of the images. Further progress for tracking the location of such organs can be achieved by using a multi-electrode configuration illustrated by way of example in figure 2.

Said multi-electrode configuration has several first electrodes 12 on a surface 14 of a breathing subject 10 as illustrated in figure 2. The outer surface electrodes 12 are defining each a unique line 22 by applying said ac voltage 18 to an intracardiac electrode 16 within the heart 13. Thus several impedances 20 are measured. As illustrated in figure 2, the monopolar body surface electrode 12 at the left side of the shown thorax 11 should be the best choice because the highest quantity of lung tissue 24 is located between said electrode 12 and the cavities in the heart 13. With several measured impedances 20 it is possible to monitor respiration motion in higher detail. Said impedances 20 are combinable to one signal 26 by calculating a weighted sum 28 of the impedances 20. The signal 26 is especially suitable for tracking locations of organs like the heart 13.

Another possible application for the method 100 is shown in figure 2. A system 50 for compensation of motion artefacts within electrophysiological data 52 caused by respiration activity has means 54 for electrical acquiring of said electrophysiological data 52 such as an ECG of a subject 10. This acquiring means 54 comprises electrodes 12' on the subject's body, which record the data 52 continuously during respiration of the subject 10. Also there is detecting means 56 for motions 32 of the subject's chest 34, which are shown in detail in figure 3. As in figure 2 in the upper right corner illustrated, said electrophysiological data 52 is compensated with said motions 32 of the chest 34 caused by the respiration activity. This activity is monitored by a method 100 of this invention, wherein the electrodes 12' are also defining an individual line 22 for observing the change of impedance 20. A circuitry 58 is deriving a change of lung volume 36 by observed change of impedance 20 and correlating said change of volume 36 with said motions 32 of the chest 34. The motion 32 is extracted as a respiration phase signal 30 as indicated in figure 2 by the circuitry 58. The respiration phase signal 30 is of lower frequency than the ECG data 52. Once the phase signal 30 is adapted to the ECG data 52 a compensation of the motion artefacts caused by the respiration activity is available.

Said respiration phase signal 30 can be displayed to a physician for example during medical procedures like an operation or during intensive care in a hospital.

The electrophysiological data 52 could also comprise electromyographical data of the heart muscle. The intracardiac electrode 16 comprises therefore pressure detection means, which measures blood flow rates with motion artefacts caused by respiration activity. The method 100 of the invention could also be used to correct these motion artefacts.

Figure 3 illustrates another use of a method 100 or a system 50 for compensation a position 38 of a medical device 40 incorporated in a patient's body. An estimated position of the medical device 40 is tracked by measuring distances 42 between two electrodes 12' and the medical device 40. Said distances 42 are corrected according to a range 44 of motions 32 caused by respiration activity. The method 100 or the system 50 comprises several intracardiac electrodes 16 within the heart 13 and they are applied to several ac voltages 18 with several frequencies to measure each impedance 20 individually. The individual impedances 20 are for example being separated by time and/or frequency multiplexing. Filtering the electrical signal measured by one of the bipolar electrodes 12' on a surface 14 of the subject 10 allows distinguishing between at least two intracardiac electrodes 16. Therefore it is possible for example to derive a lifting up and lifting down of the chest 34 caused by down and up sizing of a lung volume 36. The lung volume can be estimated by observing changes of impedance 20 between the electrodes 12' and the intracardiac electrodes 16, which are attached to the medical device 40. The medical device 40 is a catheter in this embodiment.

The above described embodiments are not intended to limit the invention, which is exclusively determined by the appended claims. The described embodiments give only an advice for practice the invention to skilled persons. The scope of the invention is not departed by technical equivalents only.

## Claims

1. Method (100) for monitoring respiration activity of a subject (10), comprising
- (102) attaching a first electrode (12) on a surface (14) of said subject (10),
- (104) locating a second electrode (16) inside a thorax (11) of said subject (10),
- (106) applying an ac voltage (18) to one of said electrodes (12, 16),
- (108) measuring an impedance (20) between said first electrode (12) and second electrodes (16), and
- (110) observing a change of impedance (20) caused by said respiration activity,
**characterized in that,**
said second electrode (16) is at least one intracardiac electrode located within a heart (13) of said subject (10), defining a line (22) between said first electrode (12) and intracardiac electrode (16) that crosses lung tissue (24) of said subject (10).

2. Method (100) according to claim 1,
**characterized in that,**
said lung tissue (24) is crossed by said line (22) with maximum length.

3. Method (100) according either one of claims 1 and 2,
**characterized in that,**
said line (22) is defined between several first electrodes (12) and one of said intracardiac electrodes (16) and each of said measuring (108) the said impedance (20) between said several first (12) and intracardiac electrodes (16) is processed by frequency filtering (112).

4. Method (100) according to any of the proceeding claims,
**characterized in that,**
said intracardiac electrodes (16) are applied to several ac voltages (18) with several frequencies to measure said impedance (20) individually.

5. Method (100) according to any of the proceeding claims,
**characterized by** calculating (114) a signal (26) with a weighted sum (28) of said impedances (20).

6. Method (100) according to any of the proceeding claims,
**characterized by** extracting (116) a respiration phase signal (30) from said impedances (20).

7. Method (100) according to any of the proceeding claims,
**characterized in that,**
said observed (110) change of impedance (20) is displayed to a physician.

8. System (50) for compensation of motion artefacts within electrophysiological data (52) caused by respiration activity, comprising
- means (54) for electrical acquiring said electrophysiological data (52) of a subject (10), comprising electrodes (12') on the subject's body, and
- detecting means (56) for motions (32) of the subject's chest (34), whereby said electrophysiological data (52) is compensated with said motions of the chest (34),
**characterized in that,**
said respiration activity is monitored by a method (100) according to any of the proceeding claims, wherein said electrodes (12') defining said line (22), and comprising
- a circuitry (58) deriving a change of lung volume (36) by observed change of impedance (20) and correlating said change of volume (36) with said motions (32) of the chest (34).

9. Use of a method (100) or a system (50) according to any of the proceeding claims, where a position (38) of a medical device (40) incorporated in a patient's body is tracked by measuring distances (42) between two electrodes (12') and the medical device (40), wherein said distances (42) are corrected according to a range (44) of said motions (32) caused by respiration activity.
